Europäisches Patentamt

European Patent Office

Office européen des brevets

(11) Publication number: **0 213 717**
**A2**

## (12) EUROPEAN PATENT APPLICATION

(21) Application number: 86305692.5

(22) Date of filing: 24.07.86

(51) Int. Cl.⁴ **A61K 33/18** , A61K 9/02 , A61K 47/00

(30) Priority: 17.08.85 GB 8520664

(43) Date of publication of application:
11.03.87 Bulletin 87/11

(84) Designated Contracting States:
AT BE CH DE FR GB IT LI LU NL SE

(71) Applicant: Euroceltique SA
122 Boulevard De La Petrusse
Luxembourg(LU)

(72) Inventor: Buxton, Ian Richard
29 Youngman Avenue
Histon, Cambs., CB4 4HP(GB)
Inventor: Leslie, Stewart Thomas
4 Babraham Road
Cambridge, CB2 2RA(GB)
Inventor: Malkowska, Sandra Therese
Antoinette
14 Abrahams Close
Landbeach Cambridge(GB)
Inventor: Marchant, Joanne
11 Gainsborough Drive
Burleigh Hill, St. Ives, Cambs.(GB)

(74) Representative: James, Stephen Richard,
Napp Pharmaceutical Group Cambridge
Science Park Milton Road
Cambridge CB4 4GW(GB)

(54) Iodophor containing suppository.

(57) A suppository containing at least one iodophor, a water soluble, suppository base, at least one water soluble sugar having a molecular weight between 90 and 550, especially between 150 and 370, and at least 30% (by wt) water.

Preferably the iodophor is povidone iodine or polydextrose iodine, the suppository base is a gelling polysaccharide and the sugar is a monosaccharide, disaccharide or sugar alcohol.

A high concentration of water, together with the presence of a low molecular sugar, improves the rate of iodine release from the suppository.

## IODOPHOR CONTAINING SUPPOSITORY

The present invention relates to suppositories, in particular to iodophor containing suppositories, especially to iodophor containing vaginal suppositories (pessaries).

Suppositories containing an iodophor, povidone iodine, are known. These suppositories consist of povidone iodine and polyethylene glycol (M.Wt. about 1000) only. Although the level of iodine activity achieved by these suppositories is acceptable, it could usefully be improved.

Such an improvement would be particularly useful in the case of iodophor containing vaginal suppositories, but may also be advantageous in the case of rectal suppositories in which antibacterial agents are added to supplement the action of, for example, anti-inflammatory agents.

In the present specification, the "level of iodine activity" refers to the concentration of equilibrium - (free) iodine at the site of iodophor action. Free iodine is responsible for the microbicidal action of iodophors.

It is therefore one object of the present invention to provide an iodophor-containing suppository, especially vaginal suppository, having an improved level of iodine activity.

Other objects and advantages of the present invention will become apparent from the following detailed description thereof.

According to the present invention there is provided a suppository comprising at least one iodophor, a water-soluble, suppository base, at least one water soluble, low molecular weight sugar and at least 30% (by wt) water. Preferably the suppository is a vaginal suppository (pessary).

Preferably the concentration of water in the suppository is between 40% and 80% (by wt.), especially between 50% and 70% (by wt.).

The present inventors have surprisingly found that by adding at least one water soluble, low molecular weight sugar and a high proportion of water to iodophor containing suppositories, the level of iodine activity is improved in comparison with the level achieved by known, anhydrous iodophor containing suppositories.

Iodophors employed in the present suppository are water-soluble, physiologically-acceptable complexes of iodine with organic polymers, in which the germicidal and microbiocidal activity of elemental iodine is maintained. Examples of such iodophors include combinations of elemental iodine with detergent polymers, such as nonylphenoxy poly (ethyleneoxy) ethanol and undecoylium chloride. In a particularly preferred embodiment of the present invention, however, the at least one iodophor is a complex of iodine with a suitable non-ionic, non-detergent (non-surface active), water-soluble organic polymer, such as polyvinylpyrrolidone (povidone), polydextrose or a copolymer of sucrose and epichlorohydrin. Of these non-ionic, non-detergent organic polymers, povidone and polydextrose are especially preferred.

Polyvinylpyrrolidone is a non-ionic, non-detergent water-soluble, organic polymer that is characterised by an unusual complexing ability, by its colloidal properties and by its physiological inertness. Its iodine complex, poly vinylpyrrolidone - (povidone) iodine, is a well known iodophor that is a highly effective germicide, providing a broad spectrum of microbiocidal action against virtually all microbes.

Povidone iodine may be prepared by any of a number of known routes, see, for example, European Published Applications No. 120301A and 6340A, and GB 1580596, the contents of which references are incorporated herein by reference.

Polydextrose is a non-nutritive polysaccharide, prepared by the condensation polymerisation of saccharides in the presence of polycarboxylic acid catalysts, under reduced pressure. Polydextrose is described in US Patents No. 3766105 and 3786794, and is available from Pfizer Inc., New York. Commercially available polydextrose polymer is a low molecular weight, water-soluble, randomly bonded polymer of glucose containing minor amounts of sorbitol end groups and citric acid residues attached to the polymer by mono-and di-ester bonds. The number average molecular weight of this commercially available material is 1,500, ranging from about 160 to about 20,000.

When polydextrose polymer is combined with elemental iodine, preferably in the presence of an alkali metal iodide, the resultant polydextrose-iodine complex is formed. This complex is a tan-to-amber coloured product which melts between 90¤C and 130¤C to form a red liquid. Polydextrose iodine powder is highly soluble in water, and at room temperature results in a reddish brown coloured aqueous solution.

The amount of iodine incorporated in the iodophors used in the present suppository will be determined by, amongst other factors, the amount of iodophor present in the suppository and the required germicidal strength of the suppository.

Preferably, iodine will constitute between 1 and 20% (by wt.), especially between 2 and 16% (by wt.), most especially between 2 and 12% (by wt.) of the iodophor dry weight.

Similarly, the concentration of iodophor in the present suppository will depend on the germicidal strength required. In addition, iodophor concentration will also be determined by the iodophor employed, the amount of iodine in the iodophor, the propensity of the iodophor to cause irritation, the rate of iodine loss (when the suppository is in use) and the length of suppository use contemplated. In order to treat most of the disorders contemplated by the present inventors the present suppository preferably contains enough iodophor to afford a concentration of available (thiosulphate titratable) iodine within the suppository of between 0.1 and 5% (by wt.), especially between 0.5 and 1.5% (by wt.).

The suppository base must be water-soluble. It must hold water whilst retaining its mechanical strength. The base is chosen so as to give a suppository that

(i) Is easily inserted into the body, without incidental tissue trauma or pain, and

(ii) After insertion, disintegrates readily, by dissolving, dispersing or, melting, thereby distributing the at least one iodophor within the vaginal or rectal cavity.

Preferably the base is also chosen so as to,

(iii) Draw moisture to the site of action within the vaginal or rectal cavity and thereby further activate the at least one iodophor.

Preferably the present suppository base comprises a water soluble gelling polymer (gelling in aqueous medium), such as gelatin, polyoxyethylene glycol, polymerised ethylene oxide derivatives of glycols, mixtures of polyoxyethylene glycols and polycyclohexoses (see Canadian patent no. 825687), soya protein and its derivatives, fatty acid glycerides, or, which is most preferred, a polysaccharide, such as agar, an alginate salt, a cellulose derivative or Carrageenan. In a particularly preferred embodiment of the present suppository, the suppository base comprises sodium alginate.

The water soluble, low molecular weight sugar will have a molecular weight between 90 and 550, especially between 150 and 370. The sugar may be a monosaccharide, such as the hexoses, glucose, fructose, galactose and mannose, a disaccharide, such as sucrose and lactose, or a sugar alcohol, such as mannitol, sorbitol and xylitol. The presence of the sugar, especially in combination with the gelling polymer base, facilitates the formation of the suppository, giving it enhanced mechanical rigidity. In addition to this property, the sugar will also draw moisture to the site of action in the vaginal or rectal cavity and thereby activate the iodophor.

The present suppository preferably further comprises at least one of,

(i) A water-soluble polyalcohol that is non-toxic, lubricating and demulcent. Examples include propylene glycol or, which is preferred, glycerol. In addition to acting as a lubricant and demulcent, the polyalcohol will also draw moisture to the site of action in the vaginal or rectal cavity and, like the sugar, activate the iodophor.

(ii) A surfactant, especially an anionic or non-ionic surfactant. The presence of a surfactant reduces the surface tackiness of suppositories, making them easier to remove from their packaging and, therefore, to handle. Examples of suitable surfactants include Tween (Trade Mark) and Cremophor (Trade Mark) surfactants, especially Cremophor RH40 (Trade Mark BASF), which is a glycerol polyethylene glycol oxystearate surfactant.

The concentration of the constituent or constituents of the present suppository base will be determined by, amongst other factors,

(a) The pH required at the site of action. This pH should be close to the pH in the vaginal or rectal cavity to be treated (to avoid irritancy) and should allow good microbiocidal activity. A pH between 3 and 8, especially between 3.5 and 6 will be acceptable for most therapeutic applications,

(b) The intended use of the suppository,

(c) The rigidity required for the suppository,

(d) The iodophor release characteristics required, and

(e) The amount of water and iodophor to be employed in the suppository.

Preferred concentrations (given as a % of total suppository weight) for the constituent or constituents of the present suppository are

Gelling polymer at least 2%, especially 2-30%

Polyalcohol 0-25%, especially 2-15%

Sugar 5-50%, especially 10-35%

Surfactant 0-10%, especially 1-5%.

Suppositories of the present type may be manufactured by the hot pour technique. This involves the pouring of the suppository composition, in fluid form, into a suitable mould, which is then cooled below the solidifation point of the composition to give a suppository of the desired size and shape. Alternative methods of suppository formation employing gelling polymers, such as agar and sodium alginate, will be known to those skilled in the art.

The precise size and shape of the suppository will be determined primarily by the use contemplated. Generally, a longitudinally shaped body, such as a cylindrical, conical, tapered or egg shape, and a dose form weight between 1 and 5gm. will be suitable for most applications.

The present suppository and processes for preparing the present suppository will now be described by way of example only.

Starting Materials

(i) Polydextrose Iodine (4% concentration)

Deionised water (31.2g) was placed in a stainless steel vessel fitted with an efficient mixer. With vigorous stirring, polydextrose (46.0g., Pfizer, Chemical Division, NY) was slowly added (in a portionwise manner). Once the polydextrose was dissolved in the water, potassium iodide (18.6g., USP) was added and the vigorous stirring was continued. The potassium iodide was allowed to dissolve to give a clear solution and then iodine - (4.2g., USP) was slowly added, once again with vigorous stirring. The stirring was continued until the amount of titratable iodine present, in samples taken from the reaction mixture, remained constant over a period of one hour. Finally the reaction mixture was filtered through a 200 mesh stainless steel rigimesh filter. The final solution was opaque and red-brown in colour. The polydextrose-iodine complex in the solution had an available (titratable) iodine content of about 4% (w/w).

(ii) Polydextrose Iodine (7% available iodine)

The procedure of Example (i) was followed except that the proportions of the components were adjusted to give a Polydextrose Iodine solution having an available iodine content of about 7% - (w/w).

Example 1

Glycerol (0.175g., BP), Cremophor RH40 (0.035g, Trade Mark) and distilled water (1.7 ml) were mixed. Mannitol (0.35g., BP) was then dissolved in the solution. Gelatin (0.7g, 250g Bloom, BP) was then dispersed in the solution, after which the solution was heated to 50¤C. Once the gelatin had dissolved, the mixture was allowed to cool to 40¤C. Polydextrose iodine solution (0.55g, Example (i) above) was thoroughly mixed into the solution, which was then dispensed into a cylindrically shaped mould. Finally, the composition was allowed to cool to ambient temperature, to produce a cylindrically shaped vaginal suppository, weighing 3.5g and containing 20mg of available iodine.

Example 2

A vaginal suppository having the following ingredients was prepared by the method described in Example 1,

|  | % (w/w) |
|---|---|
| Polydextrose iodine (7% available Iodine) | 8.163 |
| Mannitol | 15.00 |
| Gelatin | 20.00 |
| Cremophor RH40 | 1.00 |
| Glycerol | 5.00 |
| Distilled water | 50.837 |

The vaginal suppository produced weighed 3.5g. and contained 20 mg. of available iodine.

Example 3

A vaginal suppository having the following ingredients was prepared by the method described in Example 1,

|                                          | % (w/w) |
|------------------------------------------|---------|
| Povidone Iodine (11% available iodine)   | 5.195   |
| Gelatin                                  | 25.00   |
| Mannitol                                 | 10.00   |
| Glycerol                                 | 5.00    |
| Cremophor RH40                           | 2.00    |

Distilled water 52.805

The vaginal suppository produced weighed 3.5g. and contained 20 mg. of available iodine.

Example 4

Adipic acid (1.3g) was dispersed in Polydextrose iodine (8.2g., Example (ii) above). Calcium hydrogen phosphate (0.1g.) was then dispersed in the PDI/adipic acid dispersion.

Separately, sodium alginate (3.0g.; Manucol DM, Trade Mark) was dissolved in water (51.8g.), by gradually heating the water to 70¤C. Sucrose - (28.0g.), glucose (7.0g.) and sodium metaphosphate (0.6g.) were dissolved in the sodium alginate solution, again at 70¤C. The alginate solution was then cooled to 50¤C. 0.34g. of the PDI containing suspension was placed in a pre-formed foil mould. To this was added 3.16g. of the alginate solution. When filling was complete, the foil moulds were heat sealed and then cooled to 0¤ to 5¤C for 4 hours.

This process gave 28 suppositories each containing,

PDI 0.02g.

Sodium Alginate 0.105g.

Glucose 0.245g.

Sucrose 0.98g.

Sodium Metaphosphate 0.021g.

Calcium Hydrogen phosphate 0.0035g.

Adipic acid 0.0455g.

Water 2.08g.

Claims

1. A suppository comprising at least one iodophor, a water soluble, suppository base, at least one water soluble, low molecular weight sugar (molecular weight between 90 and 550, especially between 150 and 370) and at least 30% (by wt) water.

2. A suppository according to claim 1 characterised in that the concentration of water in the suppository is between 40% and 80%, especially between 50% and 70% (by wt).

3. A suppository according to either claim 1 or claim 2 characterised in that the at least one iodophor comprises povidone iodine or polydextrose iodine.

4. A suppository according to any one of claims 1 to 3 characterised in that the suppository base comprises a water soluble, gelling polysaccharide, especially agar, an alginate salt, a cellulose derivative or Carrageenan.

5. A suppository according to any one of claims 1 to 4 characterised in that the sugar comprises a monosaccharide, such as glucose, fructose, galactose or mannose, a disaccharide, such as sucrose or lactose, or a sugar alcohol, such as mannitol, sorbitol or xylitol.

6. A suppository according to any one of claims 1 to 5 characterised in that the suppository further comprises a water soluble polyalcohol, especially glycerol.

7. A suppository according to any one of claims 1 to 6 characterised in that the concentration of the sugar in the suppository is between 5% and 50%, especially between 10% and 35% (by wt).

8. A vaginal suppository according to any one of claims 1 to 7.

9. A process for the preparation of a suppository according to claim 1 comprising mixing at least one iodophor, a water soluble, suppository base, a water soluble, low molecular weight sugar and water, at a temperature sufficient to dissolve the iodophor, the base and the sugar in the water, and cooling the mixture.

Claims for the following Contracting State: AT

1. A process for the preparation of a suppository comprising mixing at least one iodophor, a water soluble, suppository base, a water soluble, low molecular weight sugar (molecular weight between 90 and 550, especially between 150 and 370) and water, at a temperature sufficient to dissolve the iodophor, the base and the sugar in the water, and cooling the mixture.

2. A process according to claim 1 <u>characterised</u> <u>in</u> <u>that</u> the concentration of water in the suppository is between 40% and 80%, especially between 50% and 70% (by wt).

3. A process according to either claim 1 or claim 2 <u>characterised</u> <u>in</u> <u>that</u> the at least one iodophor comprises povidone iodine or polydextrose iodine.

4. A process according to any one of claims 1 to 3 <u>characterised</u> <u>in</u> <u>that</u> the suppository base comprises a water soluble, gelling polysaccharide, especially agar, an alginate salt, a cellulose derivative or Carrageenan.

5. A process according to any one of claims 1 to 4 <u>characterised</u> <u>in</u> <u>that</u> the sugar comprises a monosaccharide, such as glucose, fructose, galactose or mannose, a disaccharide, such as sucrose or lactose, or a sugar alcohol, such as mannitol, sorbitol or xylitol.

6. A process according to any one of claims 1 to 5 <u>characterised</u> <u>in</u> <u>that</u> a water soluble polyalcohol, especially glycerol, is mixed with the iodophor, base, sugar and water.

7. A process according to any one of claims 1 to 6 <u>characterised</u> <u>in</u> <u>that</u> the concentration of the sugar in the suppository is between 5% and 50%, especially between 10% and 35% (by wt).

8. A process according to any one of claims 1 to 7 for the preparation of a vaginal suppository.